# EUROPEAN PATENT APPLICATION

(11) **EP 3 640 273 A1**
(43) Date of publication of application: **22.04.2020**
(21) Application number: 18306356.9
(22) Date of filing: 16.10.2018
(51) Int. Cl.: C08G 18/73, C08G 83/00, C08G 18/28, C08G 18/38, C08G 73/00, C09J 175/12, C08G 101/00

(54) **DOUBLE DYNAMIC POLYMERS**

(71) Applicant: CENTRE NATIONAL DE LA RECHERCHE SCIENTIFIQUE, 75016 Paris (FR); Université de Strasbourg, 67000 Strasbourg (FR)
(72) Inventor: GIUSEPPONE, Nicolas, 67980 HANGENBIETEN (FR); MOULIN, Emilie, 67980 HANGENBIETEN (FR); LUTZ, Pierre, 67800 BISCHEIM (FR); MCKIE, Simon, 2309 COPENHAGUE (DK); GAUTHIER, Christian, 67810 HOLTZHEIM (FR); LE HOUEROU, Vincent, 67206 ACHENHEIM (FR); RUBIN, Anne, 67202 WOLFISHEIM (FR); JACOMINE, Léandro, 67100 STRASBOURG (FR)
(74) Representative: Lavoix

(57) **Abstract**

The invention relates to polymers comprising a monomer comprising a polyurethane, a supramolecular moiety, an elastomer moiety and a functional group comprising a -C=N- link, composition comprising such polymers and a process for preparing such polymers.

The invention relates to polymers having a double dynamic polymer, said presenting adhesive properties and self-healing properties, and preferably said polymer being recyclable.

## Description

The present invention relates to a new type of polymer having a double dynamic of constitution, that is combining several supramolecular and dynamic covalent bonds.

In particular, the present invention relates to a polymer comprising a monomer comprising a polyurethane, a supramolecular moiety, an elastomer moiety and a functional group comprising a -C=N- link.

The present invention also relates to a monomer for preparing said polymer, a process for the preparation of said polymer and applications thereof.

### State of the art

Polyurethanes (PUs) have been discovered just 80 years ago and since, this rich class of polymers has found numerous applications in our everyday life with a global production of 322 million tons in 2015. For industrial applications, all PUs and their analogues are obtained by irreversible polyaddition reactions between macromolecular polyols (or polyamines and alkanolamines) and polyisocyanates:

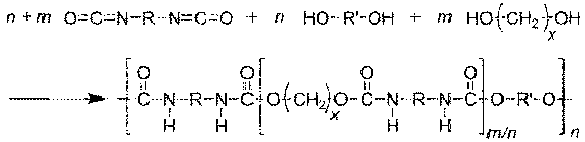

Among the most common polyisocyanates used, one will find toluene diisocyanate (TDI) and hexamethylene diisocyanate (HDI), but they can also be of higher functionality. The polyols are usually based on propylene oxide (PO) and ethylene oxide (EO) with molecular weights of 300 - 18 000 g.mol⁻¹ and functionalities comprised between 2 and 6. Branching can thus be controlled by the polyol and/or the polyisocyanate components, leading either to i) linear thermoplastic polyurethanes (TPUs) and PU elastomers, or ii) to crosslinked thermoset materials. The mechanical properties of these materials will be thus mainly dictated by the chemical structure of the prepolymers, the stoichiometry of the isocyanate/OH components, and the reaction conditions (catalysts, additives, etc.).

A large proportion (66%) of PUs raw materials are used in foam applications. Here the reaction between isocyanates and water produces carbamic acid which then decomposes into amines and carbon dioxide which serves as a blowing agent. Flexible PU foams are the materials that lead the market for cushions and mattresses; they present tunable properties such as density, hardness, elasticity and haptics. Rigid PU foams are strongly cross-linked, closed-cell materials, which can be used as structuring materials with insulating properties for construction, refrigeration, and the piping/tubing industry. For instance, in construction, and compared to panels made of polystyrene, mineral wool, or wood wool, rigid PU foams panels display much better thermal insulating properties (i.e. lower thermal conductivity). In its entire life, a PU insulating material will save 100 times the energy that has been consumed for its production.

About 20% of the PUs are used as cellular or solid elastomers for a variety of applications including shoe soles, noise vibration harshness for automotive, synthetic leathers, vibrations damping for bridges, etc. PU elastomers present good long term dynamic performance, high rebound resilience, low dynamic stiffening, high abrasion and tear resistance, and resilience to fat and oils. In addition, PUs are used in lightweight fiber composite materials in several industry sectors such as aviation and transportation in general as well as construction with a growth rate of 5% per year. They are also used as wood, metal, plastics, and textile coatings.

Another important property of PUs is their high performance adhesion. PU adhesives are supplied in solvents and water-borne dispersions which can be used directly upon drying or by addition of an excess of cross-linking isocyanate to improve heat stability. They can also be supplied as 100% systems already cross-linked and in high-molecular-weight films. The market in 2009 for PU adhesives was estimated at 2 billion euros with a volume of 640 kt (12% of the adhesive market). These adhesives are used for footwear, flexible packaging, and transportation, but also importantly for construction within concrete and wood composites. For instance the "glulam" technology involves laminated pieces of wood to produce water-resistant high-strength beams which can be curved and assembled in roof and floors. PU adhesives are also used for attaching substrates to subflooring and act as a barrier membrane to prevent water transmission. Very importantly for construction, isocyanate prepolymers are widely used as sealants which adhere to a variety of building materials with the additional property to accommodate the joint movements.

Some of key limitations and improvements are needed in PU technologies.

The effectiveness and durability of elastomeric materials over a large range of temperature are related to several technological aspects including: i) deformation recovery; ii) adhesion; iii) mechanical response to severe strains; and iv) recyclability. Deformation recovery is relatively well-understood and well-controlled. However, key improvements are currently needed for the three other aspects. For instance, good adhesion performance of polymeric materials on surfaces is of prior importance since it governs the processing in real-life applications such as spreading out, setting times, and mechanical resistance of assemblies.

This is of particular importance in construction for sealing as explained above. In addition, polymer surfaces are submitted to multiple contact strain during their lifetime, thus generating a large number of micro sliding/scratching that are difficult to repair.

PUs are often thermosets which are by essence insoluble and that cannot flow at high temperature. This causes important limitations in their recyclability, lading to very important industrial, economical, and environmental issues: it is clearly identified by academic and industrial roadmaps that new technologies are needed on that matter. Recycling technologies of thermoset PU can be divided between two categories: chemical recycling (including glycolysis, aminolysis, and thermochemical processes) and mechanical recycling (including agglomeration, grinding, and spraying). In both cases the limitation is the quality of the recycled material which is lower than the original and that are often used as additives in new formulations rather than as a fully renewed material. None of the technologies used to date is technologically and economically mature enough to afford a satisfying market for the industry, and new innovative solutions are needed to recycle thermoset PU like foams, elastomers or coatings.

As a summary, polyurethane elastomers are used for a large number of applications, going from automotive sectors to building. However, there is a clear economical need to further improve these materials towards three additional properties: adhesion, self-healing, and recyclability. Adhesion is of first importance to tightly link polyurethane elastomers to other substrates; self-healing is a needed advanced property of materials that can suffer of severe cracking; and recyclability is a major concern with important economic and environmental consequences.

### Goals of the invention

The present invention aims to solve at least one of the above described technical problems.

The present invention aims to solve the technical problem of providing a polymer having improved adhesion properties.

The present invention aims to solve the technical problem of providing a polymer having self-healing properties, i.e. reversible bonds allowing recombination after breaking.

The present invention aims to solve the technical problem of providing a polymer having self-adhesion.

The present invention aims to solve at the technical problem of providing a polymer having recyclability properties, and in particular a "reversible thermoset" material (i.e. materials that, depending on the environmental conditions can break and/or reform their covalent 3D network) and preferably presenting both enhanced mechanical properties and recyclability.

The present invention aims to solve at the technical problem of implementing simultaneously two kinds of dynamic chemical bonds (i.e. supramolecular and reversible covalent bonds with controlled relaxation times) within a polyurethane moiety.

The present invention aims to solve at the technical problem of a process for preparing such polymer.

### Detailed description of the invention

The present invention relates to a polymer comprising a monomer comprising a polyurethane, a supramolecular moiety, an elastomer moiety and a functional group comprising a -C=N- link.

In particular, said monomer comprises the following structure (I): 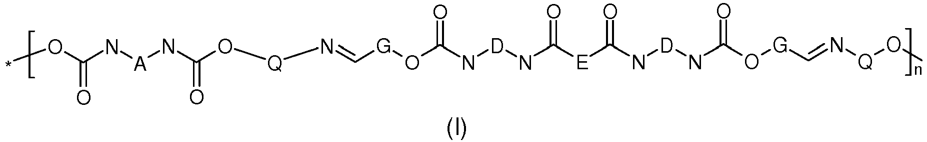 wherein:
n is the number of monomers in the polymer,
E is a chemical structure comprising a supramolecular moiety forming a supramolecular structure with a supramolecular moiety of another polymer structure (I).
A is a chemical comprising or consisting of an elastomer moiety, preferably an unsaturated elastomer,
G, D and Q are independently at each occurrence a chemical group of atoms.

### E (supramolecular moiety)

A supramolecular moiety means notably a moiety possibly forming a supramolecular structure in the presence of another supramolecular moiety by noncovalent bonds.

In one embodiment, E is selected from the group consisting of 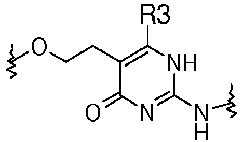 wherein R3 is selected from the group consisting of :
H; a saturated or unsaturated and linear or branched C1-C20, hydrocarbon chain which is optionally substituted and optionally interrupted by one or more heteroatoms chosen from nitrogen or oxygen or silicon or phosphorus, preferably a methyl; or a phenyl, optionally substituted, preferably: 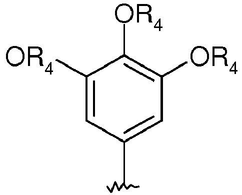 wherein R4 at each occurrence is independently selected from the group consisting of a saturated or unsaturated and linear and branched C1-C20 hydrocarbon chain which is optionally substituted and optionally interrupted by one or more heteroatoms chosen from nitrogen or oxygen or silicon or phosphorus; 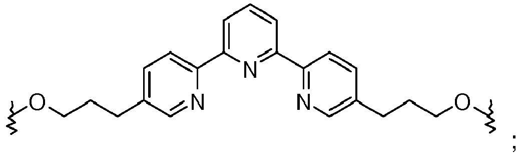 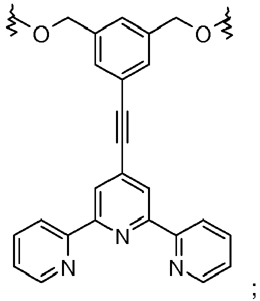 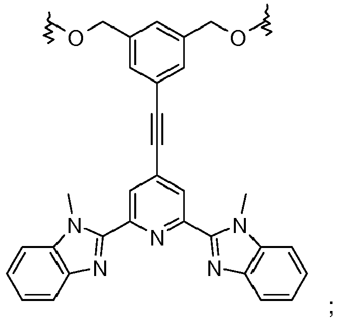 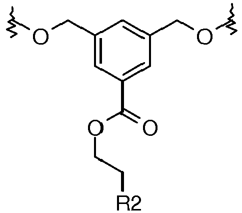 wherein R2 is selected from the group consisting of : 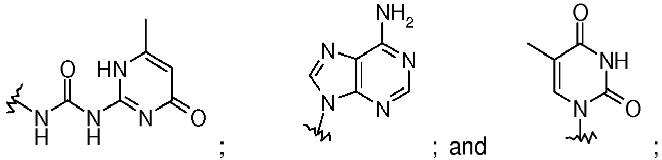 and 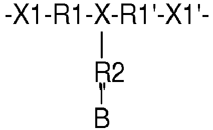
X1 and X1' denote independently an oxygen or a nitrogen atom;
X denotes a nitrogen atom, or a CR3 group where R3 is chosen from the hydrogen atom or a linear or branched C1-C20 hydrocarbon chain which is optionally substituted and optionally interrupted by one or more heteroatoms chosen from nitrogen or oxygen or silicon or phosphorus;
R2 represents a saturated or unsaturated and linear or branched C1-C20 hydrocarbon chain which is optionally substituted and optionally interrupted by one or more heteroatoms chosen from nitrogen, oxygen or silicon or phosphorus;
R1 and R1', which are identical or different, represent, independently of one another, a single bond or a saturated or unsaturated and linear or branched C1-C20, hydrocarbon chain which is optionally substituted and optionally interrupted by one or more heteroatoms chosen from nitrogen or oxygen or silicon or phosphorus;
B denotes an associative group represented by one of the structures: 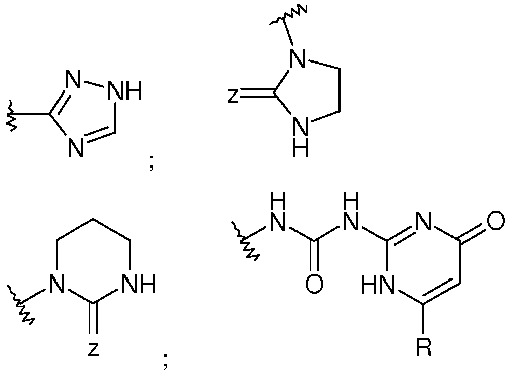 in which Z represents an oxygen or sulfur atom.

In one embodiment, 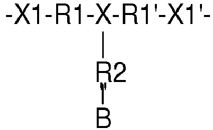 represents

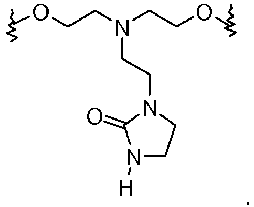

In one embodiment, E is prepared by reacting isocyanate functions with diols.

Advantageously, the diols contain a moiety forming supramolecular bonds and a functional group having a C=N function. This double dynamic cassette is at the core of the invention and allow for a convergent and systematic access to very high number of materials with finely tunable properties.

Advantageously, the reactivity of the supramolecular moiety is considered to repair localized chain cleavage.

Preferably, the supramolecular moiety is one of the parameters to control on the polymer.

It is preferred to provide a good segmental mobility, which is crucial in this type of reorganization.

A high number of dynamic polymers have been developed by implementing supramolecular recognition units covalently attached to main chain polymers and which provide plastic or rubbery properties to the initial liquid material. The kinetic lability of supramolecular bonds is attractive for self-healing properties because the recombination of the cross-link units is fast. The structures of these secondary interactions which differ from their polymer surrounding can also induce morphology changes such as aggregation and crystallization

Advantageously, different types of supramolecular units may be incorporated within the diol cassette including for instance H-bond units.

In one embodiment, E represents a group having a thymine/adenine complementary unit or diaminopyridine/uracil complementary unit. In such an embodiment two different polymer back bones are needed to provide a supramolecular structure.

In one embodiment, said supramolecular moiety comprises a hydrogen bonded polymer based on fatty diacids, triacids, amidoethyl imidazolidinone, di(aminoethyl) urea, or diamido tetraethyl urea. This leads to soft rubbers that can self-repair at ambient temperature.

In one embodiment, E comprises a ureidopyrimidinone self-complementary unit. In such an embodiment a single polymer back bone is needed to provide a supramolecular structure.

In one embodiment, E is defined by the following structure:

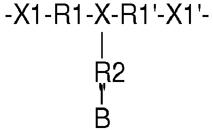

Such structure is for example defined in WO 2013/150221, including any embodiments, variants, preferred and optional features, which is incorporated herein by reference.

In one embodiment, R4 is the same at each occurrence.

In one embodiment, E represents: 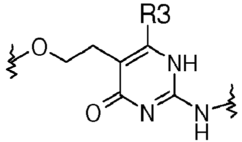 wherein R3 is alkyl, preferably methyl.

In one embodiment, said supramolecular moiety comprises metal ligand interactions to provide high binding constants by providing strong but reversible coordination polymers (also called "metallosupramolecular").

In one embodiment, said supramolecular moiety comprises poly(ethylene-co-butylene) core with 2,6-bis(1'methylbenzimidazolyl)pyridine (Mebip) which can be crosslinked by Zn(ll) ions and self-healing properties upon UV exposure.

### D, G and Q

In one embodiment, D, G and Q are at each occurrence independently selected from the group consisting of : 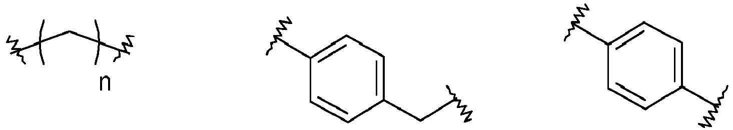 wherein the alkyl group and/or the phenyl ring are optionally substituted, preferably by one or more alkyl radicals, for example by one or more methyl radicals, wherein n is the number of CH2 is the group, preferably n is from 4 to 12.

In one embodiment, D, G and Q are at each occurrence independently a saturated or unsaturated and linear or branched C1-C20, hydrocarbon chain which is optionally substituted and optionally interrupted by one or more heteroatoms chosen from nitrogen or oxygen or silicon or phosphorus.

In one embodiment, D, G and Q are at each occurrence independently a saturated or unsaturated, linear or branched, alkyl radical, optionally substituted, preferably by one or more alkyl radicals, for example by one or more methyl radicals.

In one embodiment, D, G and Q are at each occurrence independently a saturated or unsaturated, linear or branched, C1-C20 hydrocarbon chain optionally substituted and optionally interrupted by one or more heteroatoms chosen from nitrogen or oxygen or silicon or phosphorus.

In one embodiment, D, G and Q are at each occurrence independently a linear saturated alkyl radical having 1 to 10 carbon atoms.

In one embodiment, D and/or Q are at each occurrence independently a linear saturated alkyl, preferably having from 4 to 12 carbon atoms.

In one embodiment, D and/or Q are at each occurrence independently a linear saturated alkyl having from 5 to 10 carbon atoms, for example having 6 carbon atoms.

In one embodiment, D and/or Q are at each occurrence independently selected from the group consisting of :

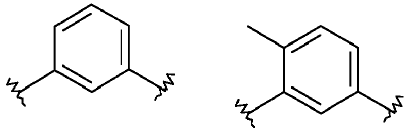

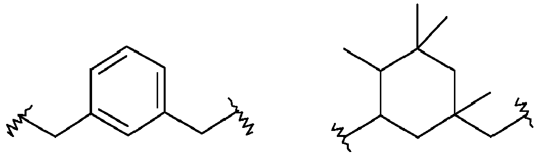

In one embodiment, D and/or Q is a phenyl, an alkylphenyl a phenylalkyl, an alkylphenylalkyl, a cycloalkyl, said phenyl, alkylphenyl, phenylalkyl, alkylphenylalkyl or cycloalkyl being optionally substituted, preferably by one or more a saturated or unsaturated, linear or branched C1-C20 hydrocarbon chain optionally substituted and optionally interrupted by one or more heteroatoms chosen from nitrogen or oxygen or silicon or phosphorus, for example said phenyl, alkylphenyl, phenylalkyl, alkylphenylalkyl or cycloalkyl being optionally substituted substituted by one or more methyl radicals.

In one embodiment, D comprises a phenyl.

In one embodiment, G and Q are at each occurrence independently a linear saturated alkyl, preferably having from 4 to 12 carbon atoms, for example having 6 carbon atoms.

In one embodiment, D comprises a phenyl and G and Q are at each occurrence independently a linear saturated alkyl, preferably having from 4 to 12 carbon atoms, for example having 6 carbon atoms.

In one embodiment, said polymer has the following structure: 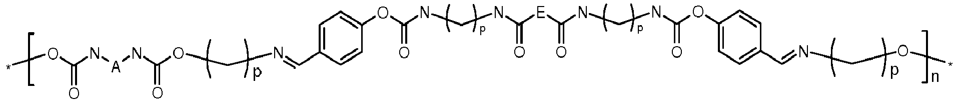 wherein A, E and n are defined according to any one of claims 2 to 7, and p is at each occurrence the same or a different index and is preferably selected from 1 to 20.

In one embodiment,p a number from 4 to 12, for example from 5 to 10, for example is 6.

### C=N function

Advantageously, the -C=N- link is a chemical function sensitive to hydrolysis.

In one embodiment, said functional group comprising a -C=N- link is selected from the group consisting of imine, oxime, hydrazine and acylhydrazone functional groups.

In one embodiment, the -C=N- link is an imine function.

In one embodiment, the -C=N- link is formed by simple condensation between a free NH₂ group and a carbonyl group. The exchange reaction of these units can be performed by hydrolysis / condensation in presence of water, but also by transamination reaction for example when a small excess of amine is present, or even cross-metathesis.

In one embodiment, the -C=N- link is hydrolyzed by pH and/or temperature variation, preferably in the presence of hydrophobic surrounding groups close to the C=N bond. The exact nature of the C=N bond itself can lead to a full variety of behavior in favor or in disfavor of the hydrolysis. Therefore the behavior regarding hydrolysis can be tuned by designing the -C=N- function.

In one embodiment, the -C=N- link is highly stable in neutral water while solubilizing in acidic pH.

Typically, but without limitation, depending on the nature of the -C=N- bond (oxime, hydrazone, acyl hydrazone, aliphatic imine, aromatic imine), the pH for hydrolysis can vary between 1 and 6.

In one embodiment, said supramolecular moiety comprises dynamic covalent bonds susceptible to re-arrange by: i) cycloaddition reactions (such as Diels-Alder), ii) exchange reactions (such as boronic esters and disulfide bonds), iii) stable free mediated reshuffling reactions (such as trithiocarbamates or thiuram disulfides), or iv) heterocyclic compounds/carbohydrate facilitated bond reformation (such as with oxetane-substituted chitosan PUs).

### A (elastomer)

In one embodiment, said elastomer is selected from the group consisting of a polyisoprene, a cis-1,4-polyisoprene, a trans-1,4-polyisoprene a polybutadiene, a chloroprene rubber, a polychloroprene, a neoprene, a baypren, copolymer of isobutylene and isoprene, an halogenated butyl rubbers, a chloro butyl rubber, a bromo butyl rubber, a copolymer of styrene and butadiene, a copolymer of butadiene and acrylonitrile, an hydrogenated nitrile rubbers, and mixture thereof.

In one embodiment, said elastomer has the following structure:

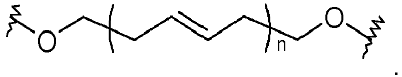

In one embodiment, the functionality of the prepolymer (bis-alcohol) can be equal to 2 (leading to thermoplastics), or >2 (leading to thermosets).

In one embodiment, the structure O(O)CN-A-NC(O)O comprises or consists of a polyurethane prepolymer (typically formed by combining an excess of diisocyanate with polyol).

In the present invention the term "alkyl" means in particular a linear or branched C1-C20 hydrocarbon chain which is optionally substituted.

Substituents can be for example one or more halogen (especially Cl, Br, F), =O, -OH or -NH2 groups.

### Monomer

The present invention relates to monomer comprising the following structure: 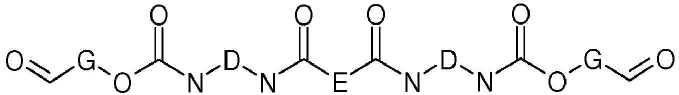 or 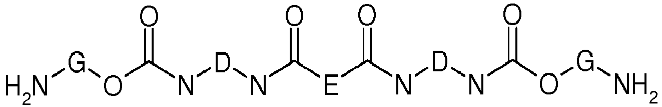 or 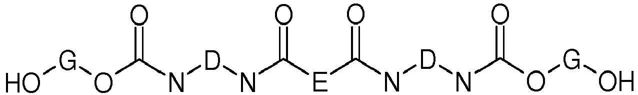 wherein B, D and E are defined according to the present invention. In one embodiment, said monomer presents the following structure: 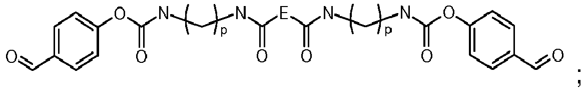 and for example presents the following structure: 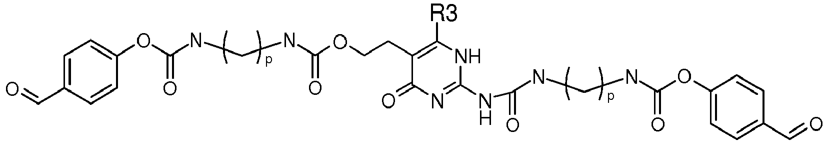 wherein R3 is selected from the group consisting of :
H, an alkyl, preferably methyl, or a phenyl, optionally substituted, preferably: 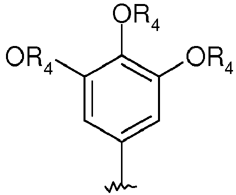 wherein R4 at each occurrence is independently at each occurrence H or a saturated or unsaturated and linear or branched C1-C20 hydrocarbon chain which is optionally substituted by one or more =O, -OH or -NH2 groups and optionally interrupted by one or more heteroatoms chosen from nitrogen, oxygen or silicon or phosphorus.

### Preparation

The present invention relates to a process for preparing a polymer, wherein said polymer is as defined according to the present invention, said polymer being prepared by reacting a monomer comprising a supramolecular moiety, said monomer being as defined according to the present invention, with a reactive molecule comprising at least two urethane functions and an elastomer moiety, said reactive molecule comprising reactive groups reacting with said monomer to form an imine function, thereby forming said polymer.

In one embodiment, said reactive molecule presents the following structure: 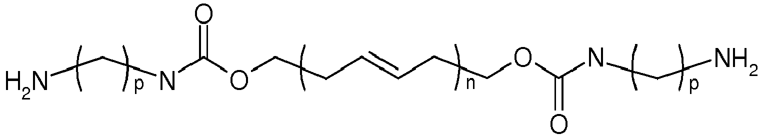 wherein p is at each occurrence the same or a different index and is preferably selected from 1 to 20.

In one embodiment, said process comprises reacting with

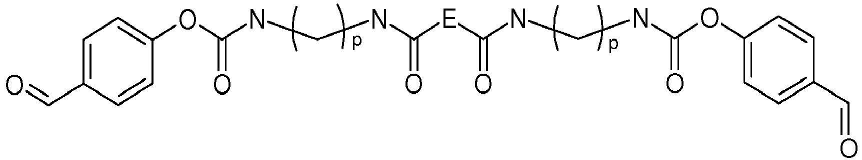

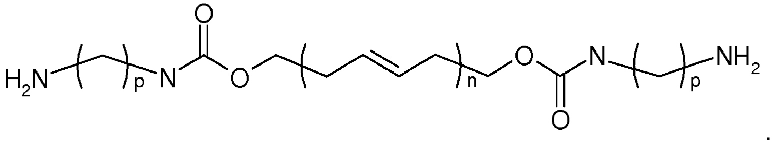

Said imine is formed by condensation of an aldehyde with an amine.

Said imine is formed by transamination of an imine and an amine.

Said imine is formed by metathesis of two imines

In one embodiment, said process comprises the chemical synthesis of a diisocyanate compound.

In one embodiment, said process comprises the chemical synthesis of a dialdehyde-functionalised molecule comprising at least one supramolecular moiety, for example starting from said diisocyanate compound. Preferably, said dialdehyde-functionalised molecule comprising at least one supramolecular moiety is a dialdehyde-functionalised ureidopyrimidinone.

In one embodiment, said dialdehyde-functionalised molecule comprising at least one supramolecular moiety has the following structure:

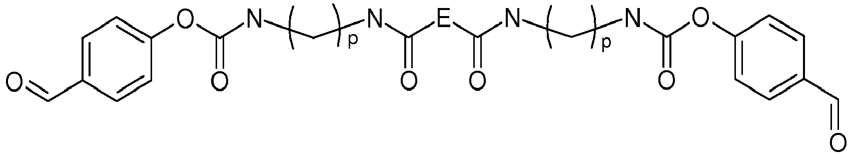

In one embodiment, said process comprises the chemical synthesis of an amine-functionalized diene-polymer, typically by reacting a diene-polymer and carbonyl diimidazole.

In one embodiment, said process comprises the chemical synthesis of a thermoplastic, for example by reacting an amine-functionalized diene-polymer with a dialdehyde-functionalised molecule comprising at least one supramolecular moiety.

In one embodiment, said process comprises the chemical synthesis of a thermoset, for example by reacting an amine-functionalized diene-polymer with a dialdehyde-functionalised molecule comprising at least one supramolecular moiety.

In one embodiment, amine modified prepolymer and aldehyde modified E moiety are solubilized in a solvent, for example chloroform (minimum quantity is preferred), typically under argon in two different flasks. The resulting solutions are then placed (for example pipetted) into a mold (typically Teflon® mold) and allowed to evaporate in an atmosphere of solvent, for example chloroform overnight, before post-curing, for example at 50°C under vacuum for 5 hours.

### Advantages

In one embodiment, said polymer is double-dynamic polyurethane elastomer by presenting adhesive properties and self-healing properties, preferably being polymer is recyclable.

Advantageously, said polymer combines different chemistries of different time scales to adapt to the whole range of the polymer reorganization dynamics.

Advantageously, said polymer enhances adhesion and self-healing properties, as well as their recyclability.

Advantageously, said polymer provides a good modularity and a variety of the proposed structures which are not limited to the ones described in the specific structures represented in the present invention.

Advantageously, said polymer is soluble and recyclable in water.

Advantageously, said polymer has improved adhesion. Such improved adhesion is for example provided by providing quickly accessible reactive groups at the surface of the polymer for example because of their reversible supramolecular exchange with the bulk material. The invention also relates to a method for improving adhesion, wherein said method implements a polymer according to the present invention. In one embodiment, said method provides self-adhesion of thermoplastics and/or thermosets upon exposure to a stimuli such as for example exposure to heat or a solvent.

In one embodiment, said polymer is mainly based on polyisoprene and polybutadiene synthetic rubbers which are known to provide good adhesion properties and outstanding mechanical properties when permanently cross-linked. Their molar mass, their microstructure, and their functionality can be precisely controlled and many of them are commercially available, already incorporated in industrial production of polyurethanes.

Advantageously, said polymer has reversible bonds allowing recombination after breaking, for example by a process induced by temperature, electromagnetic radiations, or chemical modifications such as pH, concentration, redox environment, etc.

The invention covers the functionality of the chemical structures.

### Mechanical tests

Mechanical tests were performed using the standard Dynamic Mechanical Thermal Analysis (DMTA). This technique is very discriminatory for the different materials and is of crucial interest to evaluate the dynamic response of the elastomers. The exploration is achieved throughout a large range of temperature (typically from -60 to +150 °C). Time-temperature equivalence used to draw master curves that represent the time-dependent behavior of the material on a broad time spectrum (from very slow - which will involve self-healing - to fast time scales). Strain-stress curves are achieved through tensile tests after DMTA tests on the same batch of materials so as the description of the elastomers at finite strain can be determined. These tests also permit to create an array of cracks before final failure which are studied as benchmarks for self-healing. This technique allows to extract for the pristine, self-healed or recycled materials the Young modulus, the tensile strength, and the strength at rupture of the elastomers.

In one embodiment, a polymer according to the invention presents a Young's Modulus of at least 3 MPa.

In one embodiment, a polymer according to the invention presents a Young's Modulus of at least 5 MPa, preferably at least 10MPa.

In one embodiment, a polymer according to the invention presents a Tensile Strength of at least 1 MPa, preferably at least 2 MPa.

In one embodiment, a polymer according to the invention presents a Strain at Rupture of at least 1 mm/mm.

### Recycling capabilities

In one embodiment, said polymer presents reversible covalent bonds with controlled relaxation times. By adding an activator (acid) and water, this dynamic covalent bond can hydrolyze with a speed that depends on the exact procedure, up to a full disruption of the polymer network (in thermoplastic, but also thermoset initial topology). After neutralization of the pH in biphasic condition (typically THF/water), remolding and evaporation of the solvents, the full network can be recondensed by imine formation, leading to a recycled elastomer.

### Compositions

The present invention relates to a composition comprising at least one polymer is as defined according to the present invention.

### Applications

The present invention relates to the use of a polymer is as defined according to the present invention as a double dynamic polymer, said presenting adhesive properties and self-healing properties, and preferably said polymer being recyclable.

In one embodiment, said polymer is recyclable by pH variation.

Typically the polymers or composition of the present invention can be used in the following technical fields, depending on their exact properties, notably provided by the elastomer moiety: as polyurethane foam sponge, as a polymer for manufacturing furniture, as memory foam cushions, as open cell flexible polyurethane foam, as flexible polyurethane foam including upholstered furniture cushions, automotive seat cushions and interior trim, carpet cushion, mattress padding or solid-core mattress cores, as flexible polyurethane foam, as polymer for automobile seats, as flexible or semi-flexible polyurethane foams, notably for interior components of automobiles, in seats, headrests, armrests, roof liners, dashboards or instrument panels, as a polymer for houses, sculptures, decorations, walls, ceiling, buildings, statues, as rigid foam, notably to replace wood, as thermal insulator, as polyurethane resin, for example as an aesthetic flooring material, as a polyurethane foam, as a wall insulation, as polyurethane foam for joist cavity insulation, as polymer for filling of spaces or cavities, as a polymer for construction sealants or firestopping, as a polymer for water vessels, inflatable boats, surfboards, rigid-hulled boats, boat decks or outdoor marine surface areas, as flexible plastics, as tennis grips, as polymer for watch-band wrapping, as polyurethane watch strap, as a polymer for textiles, as polyurethane fiber clothing, as varnish, as polyurethane materials for paints or varnishes, for example for finishing coats, to protect or seal wood or constructions, especially roofs, as a polymer for manufacturing wheels, automotive parts, including bumpers, side skirts, roll pans, and wiper cowls, as a polymer for manufacturing electronic components or as an adhesive.

In one embodiment, said polymer or composition containing said polymer is for use as a varnish or paint.

In one embodiment, said polymer or composition containing said polymer is for use as an adhesive.

In one embodiment, said polymer or composition containing said polymer is for use as thermoplastic waterproofing membrane for flat roofs:
In one embodiment, said polymer or composition containing said polymer is for use as waterproofing coating.

The present invention also relates to a method for recycling a material or polymer, said material or polymer comprising a polymer according to the present invention. In one embodiment, the polymer according to the present invention is recycled by pH modification, preferably in an aqueous solution, for example water. In one embodiment, the method is for recycling a thermoplastic or a thermoset material.

In the figures:

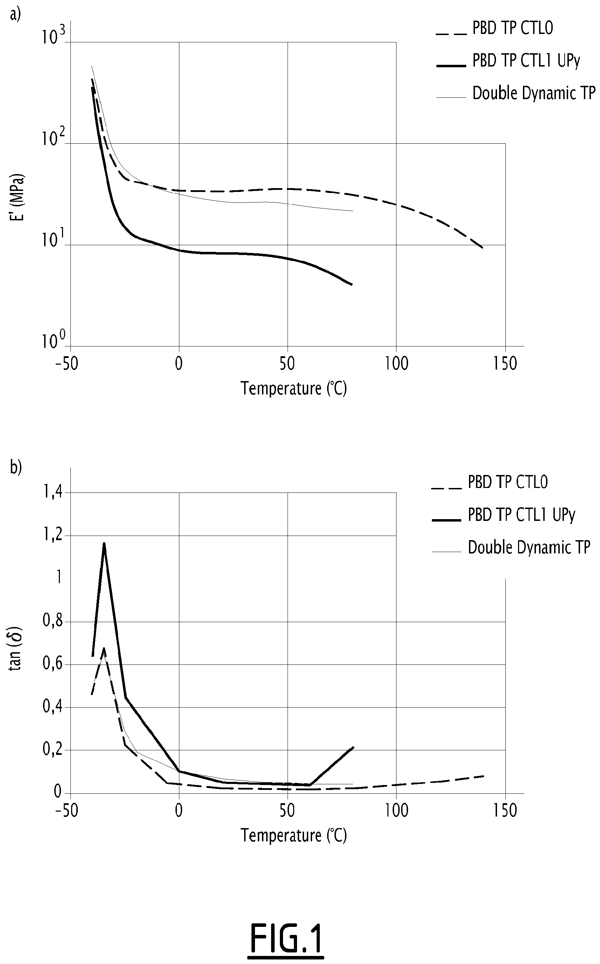

Figure 1: a) Storage modulus vs temperature, obtained by dynamic mechanical analysis, of each thermoplastic material at a frequency of 1Hz for a temperature range between -70°C and 150°C; b) Tan(δ) vs temperature, obtained by DMTA, of each thermoplastic material at a frequency of 1 Hz for a temperature range between -70°C and 150°C.

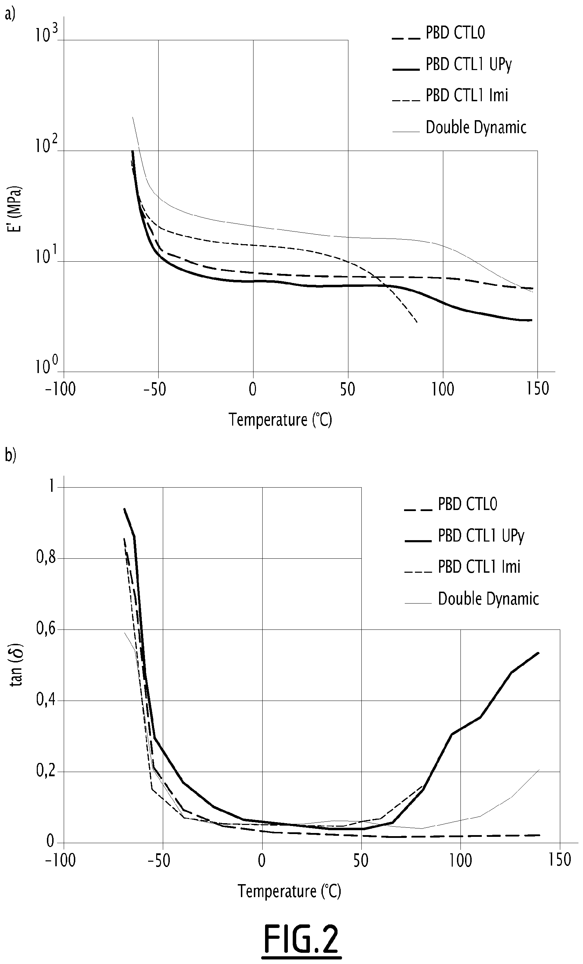

Figure 2: a) Storage modulus vs temperature, obtained by dynamic mechanical analysis, of each thermoset material at a frequency of 1Hz for a temperature range between -70°C and 150°C; b) Tan(δ) vs temperature, obtained by DMTA, of each thermoset material at a frequency of 1 Hz for a temperature range between -70°C and 150°C.

### Examples:

### Example 1 - Synthesis of polymers according to the invention

### Synthesis of dialdehyde precursor

### Synthesis of 2-(2-(3-(6-isocyanatohexyl)ureido)-6-methyl-4-oxo-1,4-dihydro pyrimidin-5-yl)ethyl (6-isocyanatohexyl)carbamate (A)

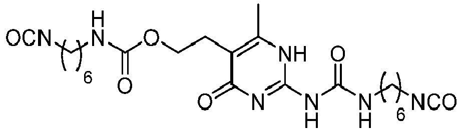

2-amino-5-(2-hydroxyethyl)-6-methylpyrimidin-4(1H)-one (2.25 g, 13.3 mmol) was added to a 2-neck round bottomed flask, which was then purged of air with three vacuum/argon cycles and filled with DMF (10 mL). To this stirred suspension, cold hexamethylene diisocyanate (HDI) (20 mL, 124.9 mmol) and pyridine (2 mL) was added; the resulting white suspension was then heated overnight at 90 ºC. Complete dissolution of the white precipitate was observed after 2 hours at heat and the clear colourless solution turned clear yellow overnight. The solution was then added drop wise to cold diethyl ether (300 mL) producing an off-white precipitate and a clear yellow liquid, which was decanted. The solid was then washed again and decanted from cold diethyl ether (2 x 300 mL) before being isolated by filtration, washed with diethyl ether (2 x 100 mL) and dried to leave compound **A** as an off-white powder (4.5 g, 67%).
¹H NMR (CDCl₃, 400 MHz, 25 ºC) δ = 12.95 (s, 1H), 11.92 (s, 1H), 10.17 (s, 1H), 4.70 (s, 1H), 4.19 (t, *J* = 6.2 Hz, 2H), 3.32 (t, *J* = 7.2 Hz, 4H), 3.29 (t, *J* = 6.8 Hz, 2H), 3.18-3.13 (m, 2H), 2.77 (t, *J* = 6.6 Hz, 2H), 2.27 (s, 3H), 1.70-1.30 (m, 16H,).
¹³C NMR (CDCl₃, 100 MHz, 25 ºC): δ = 172.2, 156.7, 156.5, 153.5, 144.9, 113.8, 62.9, 43.0, 42.9, 40.9, 39.8, 31.14, 31.09, 29.9, 29.1, 26.2, 26.1, 26.0, 25.6, 17.2.

### Synthesis of 2-(2-(3-(6-(((4-formylphenoxy)carbonyl)amino)hexyl)ureido)-6-methyl-4-oxo-1,4-dihydropyrimidin-5-yl)ethyl (4-formylphenyl) hexane- 1,6-diyldicarbamate

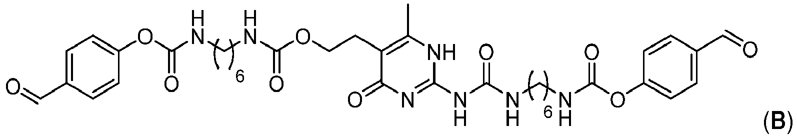

Compound **A** (1.44 g, 2.8 mmol) and 4-hydroxybenzaldehyde (1.71 g, 14 mmol) were added to a 2-necked round-bottomed flask, and purged of air by three vacuum/argon cycles. Distilled chloroform (15 mL) was added via an addition funnel and dibutyltin dilaurate (2 drops) added by syringe, via the septum, and the resulting solution stirred with heating (60ºC) overnight. The turbid orange suspension was observed to fully dissolve after 2 hours. The solution was then added drop wise to stirred diethyl ether (100 mL), the solid was then washed with ethanol (2 x 100 mL) and diethyl ether (2 x 100 mL), before drying to yield compound **B** as a white powder. (1.82 g, 87%).
¹H NMR (DMSO *d₆,* 400 MHz, 25 ºC) δ = 11.53 (bs, 1H), 9.97 (s, 2H), 9.51 (bs, 1H), 7.93 (d, *J* = 8.2 Hz, 4H), 7.33 (d, *J* = 8.2 Hz, 4H), 7.04 (t, *J* = 5.9 Hz, 1H), 3.96 (t, *J* = 6.6 Hz, 2H), 3.17-3.12 (m, 2H), 3.11-3.05 (m, 4H), 2.98-2.93 (m, 2H,), 2.61 (bt, *J* = 6.5 Hz, 2H), 2.16 (s, 3H), 1.50-1.20 (m, 16H).
¹³C NMR (DMSO *d₆,* 100 MHz, 25 ºC): δ= 191.9, 156.1, 155.9, 153.4, 132.9, 130.9, 122.2, 115.8, 29.3, 29.3, 29.2, 29.1, 29.0, 29.0, 25.9, 25.9, 25.8.

### Amine-Modification of Polybutadiene

Identical experimental procedures were used to produce amine-functionalized polybutadienes from both Polyvest® and Krasol® commercial materials. An example of the procedure, using Polyvest® is given below.

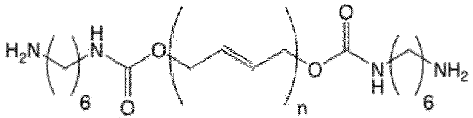

In a 2-neck round bottomed flask (with an addition funnel containing distilled chloroform and a septum attached), polybutadiene Polyvest® (3.97 g, 0.79 mmol) was added and dried under vacuum for 1 hour. The flask was then filled with argon and the polymer dissolved in chloroform (20 mL). In a separate 2-neck round bottomed flask (with a burette of distilled chloroform and a septum attached), carbonyl diimidazole (CDI) (1.29 g, 8.0 mmol) was added and purged of air with three vacuum/air cycles before being dissolved in chloroform (10 mL). The solution of polybutadiene was added drop wise to the solution of CDI and the resulting colourless solution allowed to stir overnight at room temperature. The solvent was then evaporated *in vacuo* and cyclohexane (30 mL) was added leading to the formation of a white suspension. The suspension was then filtered, washed with cyclohexane (3 x 30 mL) and the solution evaporated *in vacuo.* The resulting viscous liquid polymer was then reacted, without further purification, with 1,6-diaminohexane:
In a 2-neck round bottomed flask (with an addition funnel of distilled chloroform and a septum attached) the polymer from the previous step was purged of air by three vacuum/argon cycles. Chloroform (30 mL) was then added and the solution stirred to homogeneity before addition to of 1,6-diaminohexane (1.2 g, 10 mmol) in a chloroform solution (50 mL). The resulting solution was then stirred overnight at room temperature. The solution was then precipitated in methanol (500 mL) and the solid residue was dried *in vacuo* to yield amine-modified polybutadiene as a pale green viscous liquid polymer (3.5 g, 85%).

### Polyvest®-based Materials

¹H NMR (CDCl₃, 400 MHz, 25 °C): δ = 5.64-5.51 (br), 5.47-5.29 (br), 5.02-4.89 (br), 4.71-4.56 (br), 3.21-3.09 (br), 2.69 (t, *J* = 7.0 Hz), 2.11-1.98 (br), 1.52-1.22 (br).

### Krasol®-based Materials

¹H NMR (CDCl₃, 400 MHz, 25 °C): δ = 5.57-5.30 (br), 5.01-4.87 (br), 4.66-4.57 (br), 3.20-3.10 (br), 2.69 (t, *J* = 7.0 Hz), 2.20-1.84 (br), 1.50-1.12 (br).

### Synthesis of thermoplastics

In a three-necked round bottom flask, amine-modified Krasol®-based polybutadiene (2 g, 0.4 mmol) was added and purged of air by three vacuum/argon cycles, before addition of chloroform (5mL). In a separate flask, dialdehyde-functionalised ureidopyrimidinone (**B**) (600 mg, 0.8 mmol) was dissolved in chloroform (5 mL) and added *via* syringe to the stirred polybutadiene solution.
The resulting solution was then pipetted into a Teflon® mould and allowed to evaporate in a saturated atmosphere of chloroform, before post-curing at 50 ºC under vacuum for 5 hours.

### Synthesis of thermosets

In a three-necked round bottom flask, amine-modified Polyvest®-based polybutadiene (2 g, 0.4 mmol) was added and purged of air by three vacuum/argon cycles, before addition of chloroform (5mL). In a separate flask, dialdehyde-functionalised ureidopyrimidinone (**B**) (600 mg, 0.8 mmol) was dissolved in chloroform (5 mL) and added *via* syringe to the stirred polybutadiene solution.
The resulting solution was then pipetted into a Teflon® mould and allowed to evaporate in a saturated atmosphere of chloroform, before post-curing at 50 ºC under vacuum for 5 hours.

### Example 2 - Mechanical properties of polymers according to the invention Mechanical properties of thermoplastics

Analysis of the behaviour of the materials when stretched at very low strains was conducted using Dynamic Mechanical (Thermal) Analysis (DMTA); the materials were cut to roughly the following dimensions: 40 mm x 10 mm x 1 mm. For each of the DMTA analyses, a sinusoidal force with strains starting at 0.6%, and a peak and trough at 0.8% and 0.4% respectively, was used. The behaviour of the material was followed by observing the change in storage and loss moduli, as well as tangent delta, for a temperature range from -75 ºC to 150 ºC and using a frequency range between 0.1 Hz and 15 Hz (Figure 1a and b). The mechanical response of the double dynamic thermoplastic was compared to three different control materials: PBD TP CTL0 (amine-modified Krasol® condensed with hexamethylene diisocyanate), PBD TP CTL1 UPy (amine-modified Krasol® condensed with compound **A**), PBD TP CTL1 Imi (amine-modified Krasol® condensed with bis(4-formylphenyl) hexane-1,6-diyldicarbamate).
The limit to which the materials could be stretched until their failure, was then analyzed by elongation until break studies. All materials were tested at room temperature with a strain rate of 0.4% s⁻¹ (Table 1).

**Table 1 - Average physical parameters of the materials tested by elongation until break, over 6 experiments.**

| **Thermoplastics** | PBD TP CTL0 | PBD TP CTL1 UPy | PBD TP CTL1 Imi | Double Dynamic TP |
|---|---|---|---|---|
| Young's Modulus (MPa) | 1.65(±0.12) | 2.4(±0.22) | 2.1(±0.17) | 3.7(±0.31) |
| Tensile Strength (MPa) | 0.45(±0.15) | 0.8(±0.29) | 0.72(±0.09) | 2(±0.16) |
| Strain at Rupture (mm/mm) | 0.9(±0.08) | 0.71(±0.17) | 1.2(±0.10) | 1.44(±0.20) |

### Mechanical properties of thermosets

Analysis of the behavior of the materials when stretched at very low strains was conducted using Dynamic Mechanical (Thermal) Analysis (DMTA); the materials were cut to roughly the following dimensions: 40 mm x 10 mm x 1 mm. For each of the DMTA analyses, a sinusoidal force with strains starting at 0.6%, and a peak and trough at 0.8% and 0.4% respectively, was used. The behaviour of the material was followed by observing the change in storage and loss moduli, as well as tangent delta, for a temperature range from -75 ºC to 150 ºC and using a frequency range between 0.1 Hz and 15 Hz (Figure 2a and b). The mechanical response of the double dynamic thermoset was compared to three different control materials: PBD CTL0 (amine-modified Polyvest® condensed with hexamethylene diisocyanate), PBD CTL1 UPy (amine-modified Polyvest® condensed with compound **A**), PBD CTL1 Imi (amine-modified Polyvest® condensed with bis(4-formylphenyl) hexane-1,6-diyldicarbamate).

The limit to which the materials could be stretched until their failure, was then analyzed by elongation until break studies. All materials were tested at room temperature with a strain rate of 0.4% s⁻¹ (Table 2).

**Table 2 - Average physical parameters of the materials tested by elongation until break, over 6 experiments.**

| **Thermosets** | PBD CTL0 | PBD CTL1 UPy | PDB CTL2 Imi | Double Dynamic |
|---|---|---|---|---|
| Young's Modulus (MPa) | 2.69 (±0.34) | 4.90 (±1.35) | 2.7(±0.25) | 14.46 (±3.32) |
| Tensile Strength (MPa) | 0.94 (±0.24) | >3.10 (±0.86) | 2.3(±0.6) | >4.70 (±1.66) |
| Strain at Rupture (mm/mm) | 0.57 (±0.20) | >2.25 (±0.53) | 0.6(±0.3) | >1.12 (±0.58) |

### Example 3 - Self-healing properties of polymers according to the invention

### Self-healing properties of thermoplastics and thermosets

Two different types of cut, namely half-width and half-thickness (half-half) and half-width and full-thickness (half-full) were studied on either thermoplastics or thermosets materials. Cut samples were healed by heating during ∼30 minutes in an iron-like fashion at 50-70 °C (for thermoplastics) and at 110-120 °C (for thermosets). The samples have shown the recovery of mechanical properties close to quantitative after 5 cycles of iron-like procedure.

### Self-adhesion of thermoplastics and thermosets

Adhesion between two pieces of Double Dynamic materials was examined using the JKR model of elastic contact. The extent of self-adhesion when exposed to either of the two stimuli, heat or solvent, was investigated by mounting two pieces of Double Dynamic material (9.3 mm x 8.5 mm) onto two flat plates with 60 N of compressive force. Upon compressing the samples at 50 ºC for 17 hours an adhesive force of 7 x 10⁵ N/m² was observed for the material made from Polyvest®. With introduction of 2 drops of THF at the interface between the two samples, and compressing them for 5 hours at room temperature, an adhesive force of 2 x 10⁵ N/m² was observed for the material made from Polyvest®.

### Example 4 - Recyclable properties of polymers according to the invention

### Recyclability of thermoplastics and of thermosets

THF (6 mL) was added to the sliced polymer (500 mg) in two portions with 90 minute delay to reach full swelling. Then, trifluoroacetic acid (60 µL) and 1 drop of water was added and the mixture was left to shake for 2 hours. Then a NaHCO₃ brine solution (2 mL) was added. The biphasic mixture was shaken for 2 hours to reach pH=7 for the aqueous layer. The THF layer was dried and pipeted into the mold to evaporate the solvent at room temperature overnight. Then molds were heated at 50°C in vacuum for 8 hours. The mechanical properties of the recycled material were then further evaluated according to mechanical experiments (DMTA, elongation until break) described previously.

## Claims

1. A polymer comprising a monomer comprising a polyurethane, a supramolecular moiety, an elastomer moiety and a functional group comprising a -C=N- link.

2. The polymer according to claim 1, wherein said monomer comprises the following structure (I): 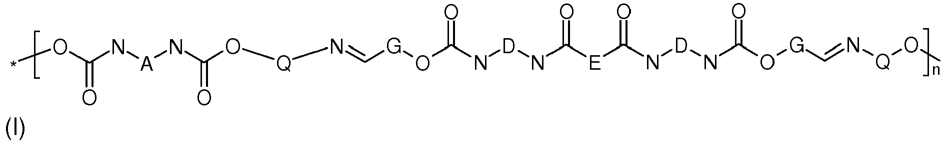 wherein:
n is the number of monomers in the polymer,
E is a chemical structure comprising a supramolecular moiety forming a supramolecular structure with a supramolecular moiety of another polymer structure (I).
A is a chemical comprising or consisting of an elastomer moiety, preferably an unsaturated elastomer,
G, D and Q are independently at each occurrence a chemical group of atoms.

3. The polymer according to claim 2, wherein E is selected from the group consisting of 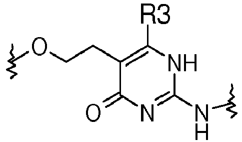 wherein R3 is selected from the group consisting of :
H; a saturated or unsaturated and linear or branched C1-C20, hydrocarbon chain which is optionally substituted and optionally interrupted by one or more heteroatoms chosen from nitrogen or oxygen or silicon or phosphorus, preferably a methyl; or a phenyl, optionally substituted, preferably: 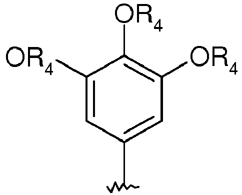 wherein R4 at each occurrence is independently selected from the group consisting of a saturated or unsaturated and linear and branched C1-C20 hydrocarbon chain which is optionally substituted and optionally interrupted by one or more heteroatoms chosen from nitrogen or oxygen or silicon or phosphorus; 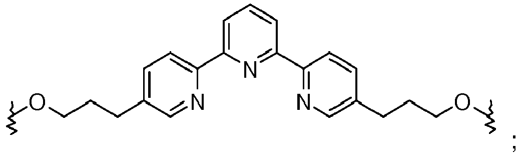 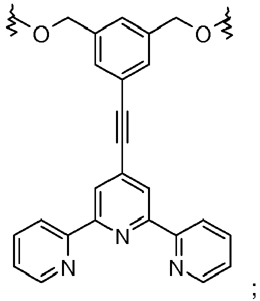 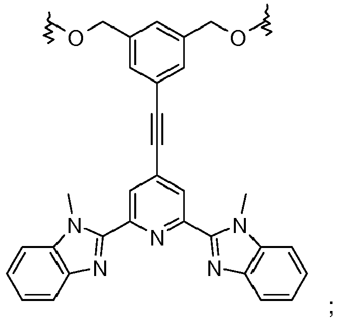 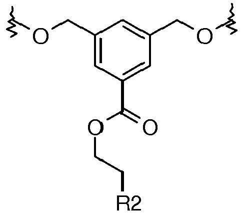 wherein R2 is selected from the group consisting of : 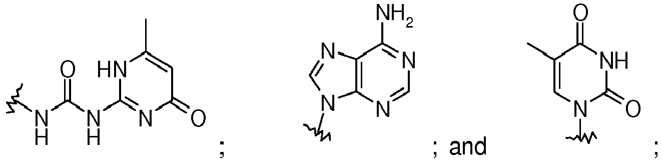 and 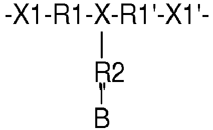
X1 and X1' denote independently an oxygen or a nitrogen atom;
X denotes a nitrogen atom, or a CR3 group where R3 is chosen from the hydrogen atom or a linear or branched C1-C20 hydrocarbon chain which is optionally substituted and optionally interrupted by one or more heteroatoms chosen from nitrogen or oxygen or silicon or phosphorus;
R2 represents a saturated or unsaturated and linear or branched C1-C20 hydrocarbon chain which is optionally substituted and optionally interrupted by one or more heteroatoms chosen from nitrogen, oxygen or silicon or phosphorus;
R1 and R1', which are identical or different, represent, independently of one another, a single bond or a saturated or unsaturated and linear or branched C1-C20, hydrocarbon chain which is optionally substituted and optionally interrupted by one or more heteroatoms chosen from nitrogen or oxygen or silicon or phosphorus;
B denotes an associative group represented by one of the structures: 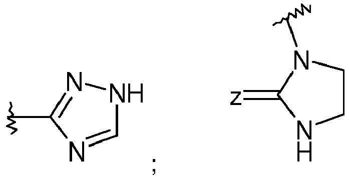 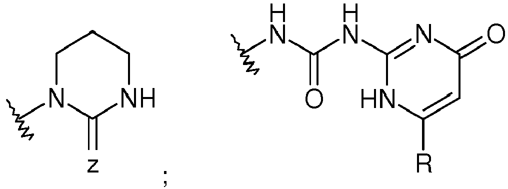 in which Z represents an oxygen or sulfur atom.

4. The polymer according to claim 2 or 3, wherein D, G and Q are at each occurrence independently selected from the group consisting of : 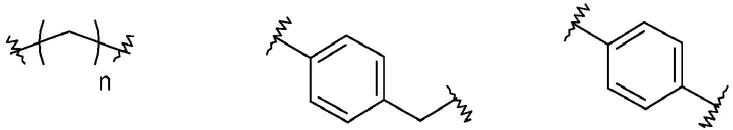 wherein the alkyl group and/or the phenyl ring are optionally substituted, preferably by one or more alkyl radicals, for example by one or more methyl radicals, wherein n is the number of CH2 is the group, preferably n is from 4 to 12.

5. The polymer according to any one of claims 2 to 4, wherein D and/or Q is a phenyl, an alkylphenyl a phenylalkyl, an alkylphenylalkyl, a cycloalkyl, said phenyl, alkylphenyl, phenylalkyl, alkylphenylalkyl or cycloalkyl being optionally substituted, preferably by one or more a saturated or unsaturated, linear or branched C1-C20 hydrocarbon chain optionally substituted and optionally interrupted by one or more heteroatoms chosen from nitrogen or oxygen or silicon or phosphorus, for example said phenyl, alkylphenyl, phenylalkyl, alkylphenylalkyl or cycloalkyl being optionally substituted substituted by one or more methyl radicals.

6. The polymer according to any one of claims 2 to 5, wherein said functional group comprising a -C=N- link is selected from the group consisting of imine, oxime, hydrazine and acylhydrazone functional groups.

7. The polymer according to any one of claims 2 to 6, wherein said elastomer is selected from the group consisting of a polyisoprene, a cis-1,4-polyisoprene, a trans-1,4-polyisoprene a polybutadiene, a chloroprene rubber, a polychloroprene, a neoprene, a baypren, copolymer of isobutylene and isoprene, an halogenated butyl rubbers, a chloro butyl rubber, a bromo butyl rubber, a copolymer of styrene and butadiene, a copolymer of butadiene and acrylonitrile, an hydrogenated nitrile rubbers, and mixture thereof.

8. The polymer according to any one of claims 2 to 7, wherein said elastomer has the following structure:

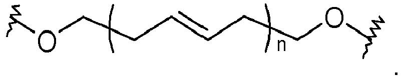

9. The polymer according to any one of claims 2 to 8, wherein said polymer has the following structure: 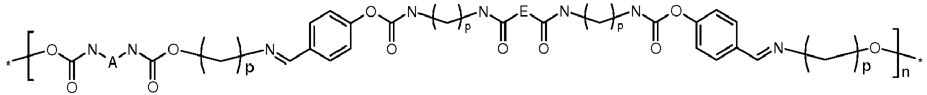 wherein A, E and n are defined according to any one of claims 2 to 7, and p is at each occurrence the same or a different index and is preferably selected from 1 to 20.

10. The polymer of any one of claims 2 to 9, wherein p a number from 4 to 12, for example from 5 to 10, for example is 6.

11. The polymer according to any one of claims 1 to 10, wherein said polymer is double-dynamic polyurethane elastomer by presenting adhesive properties and self-healing properties, preferably being polymer is recyclable.

12. A monomer comprising the following structure: 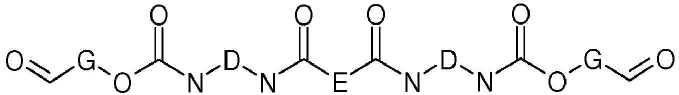 or 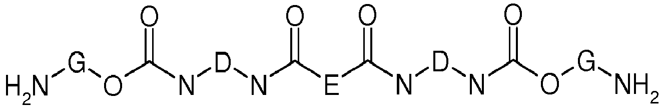 or 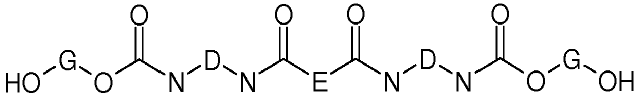 wherein B, D and E are defined according to any one of claims 2 to 8.

13. the monomer according to claim 12, wherein said monomer presents the following structure: 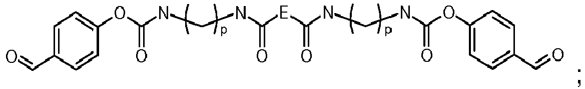 and for example presents the following structure: 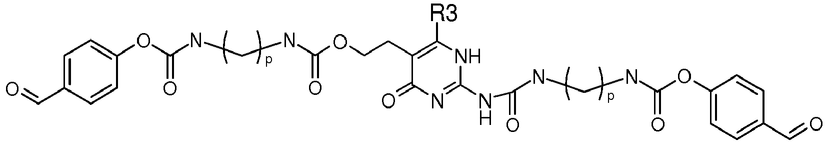 wherein R3 is selected from the group consisting of :
H, an alkyl, preferably methyl, or a phenyl, optionally substituted, preferably: 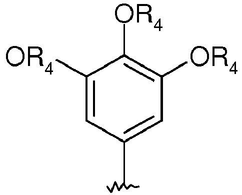 wherein R4 at each occurrence is independently at each occurrence H or a saturated or unsaturated and linear or branched C1-C20 hydrocarbon chain which is optionally substituted by one or more =O, -OH or -NH2 groups and optionally interrupted by one or more heteroatoms chosen from nitrogen, oxygen or silicon or phosphorus.

14. A process for preparing a polymer, wherein said polymer is as defined according to any one of claims 1 to 11, said polymer being prepared by reacting a monomer comprising a supramolecular moiety, said monomer being as defined according to any one of claims 12 to 13, with a reactive molecule comprising at least two urethane functions and an elastomer moiety, said reactive molecule comprising reactive groups reacting with said monomer to form an imine function, thereby forming said polymer.

15. The process according to claim 14, wherein said reactive molecule presents the following structure: 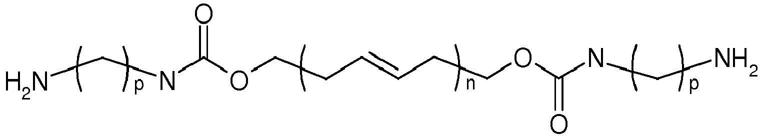 wherein p is at each occurrence the same or a different index and is preferably selected from 1 to 20.
wherein n represents the number of monomer in the elastomer moiety.

16. A composition comprising at least one polymer is as defined according to any one of claims 1 to 11.

17. The use of a polymer is as defined according to any one of claims 1 to 11 as a double dynamic polymer, said presenting adhesive properties and self-healing properties, and preferably said polymer being recyclable.

18. The use according to claim 17 wherein said polymer is recyclable by pH variation.
